(19) Europäisches Patentamt
European Patent Office
Office européen des brevets

(11) EP 0 625 928 B1

(12) EUROPÄISCHE PATENTSCHRIFT

(45) Veröffentlichungstag und Bekanntmachung des Hinweises auf die Patenterteilung:
**04.02.1998 Patentblatt 1998/06**

(21) Anmeldenummer: **94900149.9**

(22) Anmeldetag: **16.11.1993**

(51) Int. Cl.$^6$: **B01J 31/14**, B01J 31/16, C07F 5/00, B01J 31/18

(86) Internationale Anmeldenummer:
**PCT/EP93/03204**

(87) Internationale Veröffentlichungsnummer:
**WO 94/12278 (09.06.1994 Gazette 1994/13)**

(54) **KOORDINATIONS-KATALYSATORSYSTEME**
CO-ORDINATION CATALYST SYSTEM
SYSTEMES DE CATALYSEURS DE COORDINATION

(84) Benannte Vertragsstaaten:
**AT BE CH DE FR GB IT LI NL**

(30) Priorität: **01.12.1992 DE 4240294**
**03.03.1993 DE 4306569**

(43) Veröffentlichungstag der Anmeldung:
**30.11.1994 Patentblatt 1994/48**

(73) Patentinhaber: **MERCK PATENT GmbH**
**64293 Darmstadt (DE)**

(72) Erfinder:
- **POHL, Ludwig**
**D-64285 Darmstadt (DE)**
- **POETSCH, Eike**
**D-64367 Mühltal (DE)**
- **HIRSCH, Hans-Ludwig**
**D-64354 Reinheim (DE)**
- **SCHUMANN, Herbert**
**D-12623 Berlin (DE)**
- **WEISS, Karin**
**D-95447 Bayreuth (DE)**
- **THIELE, Karl-Heinz**
**D-06110 Halle (DE)**

(56) Entgegenhaltungen:
**EP-A- 0 203 286**
**EP-A- 0 231 748**
**EP-A- 0 422 590**
**EP-A- 0 432 574**
**EP-A- 0 480 247**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 625 928 B1

## Beschreibung

Die Erfindung betrifft Koordinations-Katalysatorsysteme auf Basis von Übergangsmetallverbindungen der IV. bis VIII. Nebengruppe und metallorganischen Verbindungen der III. Hauptgruppe des Periodensystems der Elemente. Derartige metallorganische Katalysatoren stellen außerordentlich vielseitige Katalysatorsysteme dar, die in chemischen Reaktionen von und mit olefinisch ungesättigten Verbindungen eingesetzt werden. Es sind dies insbesondere Verfahren zur Herstellung von Olefin-Polymeren durch Koordinationspolymerisation und die Metathese von Alkenen oder Alkinen. Von wesentlicher technischer Bedeutung ist die Herstellung von Polyethylen erhöhter Dichte (high density polyethylene, HDPE) sowie von Polymeren und Kopolymeren von Ethylen, Propylen oder anderen 1-Alkenen und Alkinen. Durch katalysierte Metathese können aus unsymetrischen Alkenen bzw. Alkinen höhere ungesättigte Kohlenwasserstoffverbindungen gezielt hergestellt und aus ungesättigten zyklischen Kohlenwasserstoffverbindungen langkettige ungesättigte Kohlenwasserstoffe erhalten werden. Letztere finden beispielsweise Anwendung bei der Herstellung von Elastomeren. Darüberhinaus finden Koordinations-Katalysatoren in weiteren Reaktionen Anwendung, wie etwa bei der Alkenhydrierung oder in metallorganischen Synthesen.

Gemäß den bisherigen wissenschaftlichen Erkenntnissen zum Wirkungsmechanismus von Koordinations-Katalysatoren wird davon ausgegangen, daß jeweils eine Übergangsmetallverbindung das katalytisch aktive Zentrum bildet, an das die olefinisch ungesättigte Verbindung in einem ersten Schritt koordinativ anbindet. Die Olefinpolymerisation erfolgt über eine Koordination der Monomeren und eine nachfolgende Insertionsreaktion in eine Übergangsmetall-Kohlenstoff- oder eine Übergangsmetall-Wasserstoff-Bindung. Die Anwesenheit von metallorganischen Verbindungen in den Koordinations-Katalysatorsystemen bzw. während der katalysierten Reaktion ist erforderlich, um den Katalysator durch Reduktion und gegebenenfalls Alkylierung oder Bildung eines komplexen Systems zu aktivieren bzw. seine Aktivität aufrechtzuerhalten. Diese Verbindungen werden daher auch als Co-Katalysatoren bezeichnet. Die das katalytisch aktive Übergangsmetallatom enthaltende Verbindung wird als Primär- oder Präkatalysator bezeichnet.

Die bekanntesten industriell angewendeten Katalysatorsysteme für die Koordinationspolymerisation sind vom Typ der "Ziegler-Natta-Katalysatoren" und vom Typ der "Phillips-Katalysatoren". Erstere bestehen aus dem Reaktionsprodukt eines Metallalkyls oder -hydrids der Elemente der ersten drei Hauptgruppen des Periodensystems und einer reduzierbaren Verbindung eines Übergangsmetallelements der IV. bis VII. Nebengruppe, wobei die am häufigsten angewendete Kombination aus einem Aluminiumalkyl, wie Aluminiumtriethyl oder Diethylaluminiumchlorid, und Titan(IV)chlorid besteht. Neuere hochaktive Ziegler-Natta-Katalysatoren stellen Systeme dar, in denen die Titanverbindung chemisch an der Oberfläche von Magnesiumverbindungen, wie insbesondere Magnesiumchlorid, fixiert ist.

Als Phillips-Katalysator kommen auf anorganischen Trägern gebundene Chromverbindungen zum Einsatz, die eine Reduktion bzw. Aktivierung vornehmlich durch metallorganische Verbindungen erfahren. Als katalytisch aktive Spezies werden Cr(VI) und Cr(II) angesehen ("reduzierter Phillips-Katalysator"). Als Co-Katalysatoren kommen auch hier vornehmlich Aluminiumalkylverbindungen sowie Alumoxanverbindungen zum Einsatz.

Neuere Entwicklungen zu besonders leistungsfähigen Polymerisationskatalysatoren richten sich auf Metallocen-Verbindungen. Unter der Bezeichnung "Kaminsky-Katalysatoren" sind beispielsweise Titanocen- und Zirkonocen -Verbindungen bekannt, die Cyclopentadienylkomplexe von Titan- bzw. Zirkonalkylen oder -halogeniden sowie Derivate hiervon darstellen, die mit Hilfe von Aluminium-, Bor- oder Phosphortrialkylen oder Alumoxan aktiviert werden.

Die praktische Anwendung dieser Katalysatoren und verwandter Typen in den in großer Vielfalt entwickelten Verfahrensvarianten kann Produkte mit zum Teil höchst unterschiedlichen Eigenschaften liefern. Bei Olefin-Polymeren, die als Werkstoffe von allgemein bekannter Bedeutung sind, richten sich Einsetzbarkeit und Einsatzbereich eigenschaftsbedingt zum einen nach der Art der zugrundeliegenden Monomere bzw. nach Auswahl und Verhältnis der Comonomere und den, das Polymer charakterisierenden typischen physikalischen Parametern, wie mittlere Molmasse, Molmassenverteilung, Verzweigungsgrad, Vernetzungsgrad, Kristallinität, Dichte, Anwesenheit funktioneller Gruppen im Polymer usw., zum anderen nach verfahrenstechnisch bedingten Eigenschaften, wie Gehalt an niedermolekularen Verunreinigungen, Anwesenheit von Katalysatorresten und letztendlich nach den Kosten.

Zur Beurteilung der Leistungsfähigkeit eines Koordinations-Katalysatorsystems sind neben der Verwirklichung der gewünschten Produkteigenschaften weitere Faktoren entscheidend, wie die Aktivität des Katalysatorsystems, also die für eine wirtschaftliche Umsetzung einer vorgegebenen Menge Olefin erforderliche Menge an Katalysator, der Produktumsatz pro Zeiteinheit und die Produktausbeute, der Verlust an Katalysator sowie die Wiederverwendbarkeit des Katalysators. Es werden daher Katalysatorsysteme mit einer möglichst hohen Produktivität aber auch mit hoher Spezifität zugunsten eines geringen Verzweigungsgrades und einer hohen Stereoregularität des Polymers gesucht, wobei letzteres insbesondere für Polypropylen und Polymere höherer 1-Alkene wichtig ist.

Wesentlich ist aber auch die Frage der Stabilität und der Handhabbarkeit des Katalysators bzw. seiner Komponenten. Praktisch alle bekannten Koordinations-Katalysatoren sind äußerst luft- und feuchtigkeitsempfindlich. Durch Zutritt von (Luft-)Sauerstoff und/oder Wasser werden die bekannten Koordinations-Katalysatoren in ihrer Aktivität vermindert oder irreversibel zerstört. Reduzierte Phillips-Katalysatoren, beispielsweise, glühen bei Luftzutritt sofort auf und sind dann unbrauchbar. Die Koordinations-Katalysatoren müssen daher bei Herstellung, Lagerung und Einsatz strikt vor

Luft- und Feuchtigkeitszutritt bewahrt werden, was naturgemäß die Handhabung erschwert und den erforderlichen Aufwand erhöht.

Übliche Katalysatorsysteme sind auch empfindlich gegenüber Stoffen, die elektronenreiche Elemente wie etwa Sauerstoff oder Stickstoff enthalten. Verbindungen wie Alkohole und Amine oder auch polare Monomere, die als Comonomere oder Additive für das Polymer von Interesse sein können, desaktivieren den Katalysator.

Noch empfindlicher in dieser Hinsicht und daher noch schwieriger handhabbar sind die als Aktivatoren bzw. Cokatalysatoren einzusetzenden metallorganischen Verbindungen, wie insbesondere die überwiegend hierfür verwendeten Aluminumalkylverbindungen. Gerade diese stellen aufgrund ihrer extremen Empfindlichkeit und Selbstentzündlichkeit in der Praxis ein ernstes Problem dar.

Es bestand daher ein besonderes Bedürfnis, weniger empfindliche metallorganische Verbindungen aufzufinden, die sich aber trotzdem als aktiverende Komponenten in Koordinations-Katalysatorsystemen eignen. Mit diesen Ersatzverbindungen sollten Koordinations-Katalysatorsysteme mit zumindest gleichen Anwendungseigenschaften und möglichst noch größerer Einsatzbreite zugänglich gemacht werden können. Diese sollten selbst wiederum eine geringere Empfindlichkeit und damit eine problemlosere Handhabbarkeit aufweisen.

Es wurde nun gefunden, daß sich zyklische metallorganische Verbindungen der Formeln I oder II

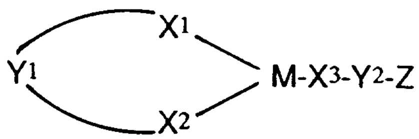

(I)

worin

| | |
|---|---|
| M | B, Al, Ga, In, |
| $X^1$, $X^2$, $X^3$ | jeweils unabhängig voneinander $CHR^1$, $NR^2$, O, S |
| $Y^1$, $Y^2$ | jeweils unabhängig voneinander $-(CH_2)_m-$,<br>$o-(CH_2)_p-C_6H_4-(CH_2)_q-$,<br>$o-(CH_2)_p-C_6H_6-(CH_2)_q-$,<br>$o-(CH_2)_p-C_6H_8-(CH_2)_q-$,<br>$o-(CH_2)_p-C_6H_{10}-(CH_2)_q-$,<br>$o-(CH_2)_p-C_5H_4-(CH_2)_q-$,<br>$o-(CH_2)_p-C_5H_6-(CH_2)_q-$,<br>$o-(CH_2)_p-C_5H_8-(CH_2)_q-$,<br>$-(CH_2)_p-CH{=}CH-(CH_2)_q-$, |
| Z | $NR^3R^4$, $PR^3R^4$, $OR^5$, $SR^5$ |
| $R^1$ | H, OH, Halogen, $C_{1-6}$-Alkyl oder $C_{1-6}$-Alkoxy, $C_{5-7}$-Cycloalkyl, Phenyl, |
| $R^2$, $R^3$, $R^4$, $R^5$ | jeweils unabhängig voneinander H oder $C_{1-6}$-Alkyl, $C_{5-7}$-Cycloalkyl, Phenyl, $R^3$ und $R^4$ zusammen auch eine $C_{4-6}$-Alkylenbrücke, |
| m | die Zahlen 1 bis 6, |
| p, q | jeweils unabhängig voneinander die Zahlen 0 bis 2 bedeuten, |

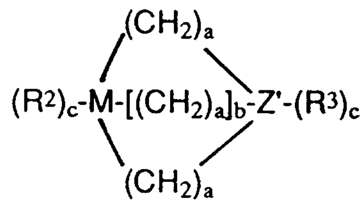

(II)

mit den vorstehenden Bedeutungen für M, $R^2$ und $R^3$ und worin

Z' N, P,

a die Zahlen 2 bis 4,

b, c die Zahlen 0 bis 1 mit b + c = 1 bedeuten,

vorzüglich als Komponenten in Koordinations-Katalysatorsystemen eignen.

Gegenstand der Erfindung ist demnach die Verwendung von zyklischen metallorganischen Verbindungen der Formeln I oder II als Komponenten in Koordinations-Katalysatorsystemen.

Gegenstand der Erfindung ist insbesondere die Verwendung dieser Verbindungen als Komponenten in Koordinations-Katalysatorsystemen für die Koordinationspolymerisation und die Metathese von Alkenen und Alkinen.

Gegenstand der Erfindung sind weiterhin Koordinations-Katalysatorsysteme auf Basis von Übergangsmetallverbindungen der IV. bis VIII. Nebengruppe und metallorganischen Verbindungen der III. Hauptgruppe des Periodensystems der Elemente, wobei diese mindestens eine Verbindung der Formel I oder II enthalten.

Gegenstand der Erfindung sind darüberhinaus Verfahren zur Herstellung von Polymeren durch Koordinationspolymerisation sowie zur Herstellung von ungesättigten Kohlenwasserstoffverbindungen durch katalysierte Metathese-Reaktion, bei denen Koordinations-Katalysatorsysteme eingesetzt werden, die mindestens eine Verbindung der Formel I oder II enthalten.

Die Verbindungen der Formeln I und II weisen eine zyklische Struktur auf, bei denen das Gruppe-IIIa-Element Bor (B), Aluminium (Al), Gallium (Ga) und Indium (In) in jedem Falle das Glied eines Ringsystems darstellt.

In Formel I bildet das Gruppe-IIIa-Element M zusammen mit den Gruppen $X^1$, $Y^1$ und $X^2$ einen Metalla-zyklischen Ring. Im einfachsten Fall wird der Ring durch eine Alkylengruppe mit insgesamt 3 bis 8 C-Atomen geschlossen. Die zu M benachbarten Gruppen $X^1$ und $X^2$ können eine Aminogruppe, Sauerstoff oder Schwefel sein. In anderen Fällen beinhaltet $Y^1$ einen aromatischen, einen aliphatischen oder einen ungesättigt aliphatischen Ring mit 5 oder 6 C-Atomen und ortho-ständiger Verknüpfung oder eine C-C-Doppelbindung mit vorzugsweise cis-Konfiguration. Ein zum Metallatom benachbartes C-Atom kann auch einen Substituenten $R^1$ tragen, der OH, Halogen, wie insbesondere F, Cl, und Br, $C_{1-6}$-Alkyl, $C_{1-6}$-Alkoxy, $C_{5-7}$-Cycloalkyl oder Phenyl sein kann. Ein derart substituiertes C-Atom stellt dann ein Chiralitätszentrum im Molekül dar. In solchem Fall repräsentiert Formel I das racemische Gemisch wie auch die entsprechenden Enantiomere bzw. Diastereomere in ihren reinen Formen. Die dritte Valenz des Metallatoms M trägt, über eine Spacer-Gruppierung $X^3$-$Y^2$ verknüpft, eine ein Heteroatom enthaltende Gruppe Z. $X^3$ kann eine $CH_2$-Gruppe, O, S oder eine gegebenenfalls mit $C_{1-6}$-Alkyl, $C_{5-7}$-Cycloalkyl oder Phenyl substituierte Aminogruppe sein. $Y^2$ ist im einfachsten Fall eine Alkylengruppe mit 1 bis 6 C-Atomen. In den anderen Fällen beinhaltet $Y^2$ einen aromatischen, einen aliphatischen oder einen ungesättigt aliphatischen Ring mit 5 oder 6 C-Atomen und ortho-ständiger Verknüpfung oder eine C-C-Doppelbindung mit vorzugsweise cis-Konfiguration. Z beinhaltet die Heteroatome N, P, O oder S in Form einer Amino-, einer Phosphino-, Hydroxy- oder Thiolgruppe, jeweils gegebenenfalls mit $C_{1-6}$-Alkyl, wobei zwei solcher Alkylreste auch eine Alkylenbrücke bilden können, oder $C_{5-7}$-Cycloalkyl oder Phenyl substituiert.

In Formel II bildet das Metallatom M über zwei oder drei Alkylenbrücken, die jeweils 2 bis 4 C-Atome enthalten können, zusammen mit dem Heteroatom Z', das N oder P sein kann, ein mono- oder bizyklisches Ringsystem. Im Falle eines monozyklischen Ringsystems sind die jeweils noch freien Bindungen von M und Z' durch H oder $C_{1-6}$-Alkyl, $C_{5-7}$-Cycloalkyl oder Phenyl abgesättigt.

Die Verbindungen der Formel I und II sind intramolekular stabilisiert durch Elektronenübertragung von dem Stickstoff-, Phosphor-, Sauerstoff- oder Schwefelatom auf das elektronenarme IIIa-Element. Sie besitzen daher im Vergleich zu üblichen Metallalkylen eine hohe Stabilität gegenüber (Luft-) Sauerstoff und Feuchtigkeit. Sie sind nicht selbst-entzündlich und damit einfach zu handhaben. Diese Stabilisierung ist auf die intramolekulare koordinative Bindung zurückzuführen.

Typische Beispiele von Verbindungen der Formel I sind:

1-Alumina-1-(4-dimethylaminobutyl)-cyclobutan
1-Alumina-1-(2-dimethylaminoethyl)-cyclopentan
1-Alumina-1-(2-diethylaminoethyl)-cyclopentan
1-Alumina-1-(2-dipropylaminoethyl)-cyclopentan
1-Alumina-1-(2-di-isopropylaminoethyl)-cyclopentan
1-Alumina-1-(2-dibutylaminoethyl)-cyclopentan
1-Alumina-1-(3-dimethylaminopropyl)-cyclopentan
1-Alumina-1-(3-diethylaminopropyl)-cyclopentan
1-Alumina-1-(3-dipropylaminopropyl)-cyclopentan
1-Alumina-1-(3-di-isopropylaminopropyl)-cyclopentan
1-Alumina-1-(3-dibutylaminopropyl)-cyclopentan
1-Alumina-1-(4-dimethylaminobutyl)-cyclopentan
1-Alumina-1-(4-diethylaminobutyl)-cyclopentan
1-Alumina-1-(4-dipropylaminobutyl)-cyclopentan
1-Alumina-1-(4-di-isopropylaminobutyl)-cyclopentan
1-Alumina-1-(4-dibutylaminobutyl)-cyclopentan
1-Alumina-1-(3-dimetylaminopropyl)-2-methyl-cyclopentan
1-Alumina-1-(2-dimethylaminoethyl)-cyclohexan
1-Alumina-1-(2-diethylaminoethyl)-cyclohexan
1-Alumina-1-(2-dipropylaminoethyl)-cyclohexan
1-Alumina-1-(2-di-isopropylaminoethyl)-cyclohexan
1-Alumina-1-(2-dibutylaminoethyl)-cyclohexan
1-Alumina-1-(3-dimethylaminopropyl)-cyclohexan, Kp. 70-73 °C/1 Pa (0,01 mbar)
1-Alumina-1-(3-diethylaminopropyl)-cyclohexan, Kp. 98 °C/60 Pa (0,6 mbar)
1-Alumina-1-(3-dipropylaminopropyl)-cyclohexan
1-Alumina-1-(3-di-isopropylaminopropyl)-cyclohexan
1-Alumina-1-(3-dibutylaminopropyl)-cyclohexan
1-Alumina-1-(4-dimethylaminobutyl)-cyclohexan
1-Alumina-1-(4-diethylaminobutyl)-cyclohexan
1-Alumina-1-(4-dipropylaminobutyl)-cyclohexan
1-Alumina-1-(4-di-isopropylaminobutyl)-cyclohexan
1-Alumina-1-(4-dibutylaminobutyl)-cyclohexan
1-Alumina-1-(o-diethylaminobenzyl)-cyclopentan
1-Alumina-1-(o-diethylaminobenzyl)-cyclohexan
1-Alumina-1-(o-di-isopropylaminobenzyl)-cyclohexan
1-Alumina-1-(3-dimethylaminopropyl)-2-methyl-cyclohexan
1-Alumina-1-(2-o-dimethylaminophenylethyl)-cyclopentan
1-Alumina-1-(2-o-diethylaminophenylethyl)-cyclobutan
1-Galla-1-(3-dimethylaminopropyl)-cyclobutan
1-Galla-1-(2-dimethylaminoethyl)-cyclopentan
1-Galla-1-(3-dimethylaminopropyl)-cyclopentan
1-Galla-1-(2-dimethylaminoethyl)-cyclopentan
1-Galla-1-(2-diethylaminoethyl)-cyclopentan
1-Galla-1-(2-dipropylaminoethyl)-cyclopentan
1-Galla-1-(2-di-isopropylaminoethyl)-cyclopentan
1-Galla-1-(2-dibutylaminoethyl)-cyclopentan
1-Galla-1-(3-diethylaminopropyl)-cyclopentan
1-Galla-1-(3-dipropylaminopropyl)-cyclopentan
1-Galla-1-(3-di-isopropylaminopropyl)-cyclopentan
1-Galla-1-(3-dibutylaminopropyl)-cyclopentan
1-Galla-1-(4-dimethylaminobutyl)-cyclopentan
1-Galla-1-(4-diethylaminobutyl)-cyclopentan
1-Galla-1-(4-dipropylaminobutyl)-cyclopentan
1-Galla-1-(4-isopropylaminobutyl)-cyclopentan
1-Galla-1-(4-dibutylaminobutyl)-cyclopentan

1-Galla-1-(3-dimethylaminopropyl)-cyclohexan, Kp. 67 °C/10 Pa (0,1 mbar)
1-Galla-1-(3-diethylaminopropyl)-cyclohexan, Kp. 94 °C/1 Pa (0,01 mbar)
1-Galla-1-(3-dipropylaminopropyl)-cyclohexan
1-Galla-1-(3-di-isopropylaminopropyl)-cyclohexan
1-Galla-1-(3-dibutylaminopropyl)-cyclohexan
1-Galla-1-(2-dimethylaminoethyl)-cyclohexan
1-Galla-1-(2-diethylaminoethyl)-cyclohexan
1-Galla-1-(2-dipropylaminoethyl)-cyclohexan
1-Galla-1-(2-di-isopropylaminoethyl)-cyclohexan
1-Galla-1-(2-dibutylaminoethyl)-cyclohexan
1-Galla-1-(4-dimethylaminobutyl)-cyclohexan, Kp. 138 °C/1 Pa (0,01 mbar)
1-Galla-1-(4-diethylaminobutyl)-cyclohexan
1-Galla-1-(4-dipropylaminobutyl)-cyclohexan
1-Galla-1-(4-isopropylaminobutyl)-cyclohexan
1-Galla-1-(4-dibutylaminobutyl)-cyclohexan
1-Galla-1-(o-dimethylaminobenzyl)-cyclobutan
1-Galla-1-(o-dimethylaminobenzyl)-cyclopentan
1-Galla-1-(o-dimethylaminobenzyl)-cyclohexan
1-Galla-1-(o-diethylaminobenzyl)-cyclohexan
1-Galla-1-(o-dipropylaminobenzyl)-cycloheptan
1-Inda-1-(2-diethylaminoethyl)-cyclobutan
1-Inda-1-(2-dimethylaminoethyl)-cyclopentan
1-Inda-1-(2-diethylaminoethyl)-cyclopentan
1-Inda-1-(2-dipropylaminoethyl)-cyclopentan
1-Inda-1-(2-di-isopropylaminoethyl)-cyclopentan
1-Inda-1-(2-dibutylaminoethyl)-cyclopentan
1-Inda-1-(3-dimethylaminopropyl)-cyclopentan
1-Inda-1-(3-diethylaminopropyl)-cyclopentan
1-Inda-1-(3-dipropylaminopropyl)-cyclopentan
1-Inda-1-(3-di-isopropylaminopropyl)-cyclopentan
1-Inda-1-(3-dibutylaminopropyl)-cyclopentan
1-Inda-1-(4-dimethylaminobutyl)-cyclopentan
1-Inda-1-(4-diethylaminobutyl)-cyclopentan
1-Inda-1-(4-dipropylaminobutyl)-cyclopentan
1-Inda-1-(4-di-isopropylaminobutyl)-cyclopentan
1-Inda-1-(4-dibutylaminobutyl)-cyclopentan
1-Inda-1-(2-dimethylaminoethyl)-cyclohexan
1-Inda-1-(2-diethylaminoethyl)-cyclohexan
1-Inda-1-(2-dipropylaminoethyl)-cyclohexan
1-Inda-1-(2-di-isopropylaminoethyl)-cyclohexan
1-Inda-1-(2-dibutylaminoethyl)-cyclohexan
1-Inda-1-(3-dimethylaminopropyl)-cyclohexan
1-Inda-1-(3-diethylaminopropyl)-cyclohexan
1-Inda-1-(3-dipropylaminopropyl)-cyclohexan
1-Inda-1-(3-di-isopropylaminopropyl)-cyclohexan
1-Inda-1-(3-dibutylaminopropyl)-cyclohexan
1-Inda-1-(4-dimethylaminobutyl)-cyclohexan
1-Inda-1-(4-diethylaminobutyl)-cyclohexan
1-Inda-1-(4-dipropylaminobutyl)-cyclohexan
1-Inda-1-(4-di-isopropylaminobutyl)-cyclohexan
1-Inda-1-(4-dibutylaminobutyl)-cyclohexan
1-Inda-1-(o-diisopropylaminobenzyl)-cyclobutan
1-Inda-1-(o-dimethylaminobenzyl)-cyclopentan
1-Inda-1-(o-dibutylaminobenzyl)-cyclopentan
1-Inda-1-(o-dimethylaminobenzyl)-cyclohexan
1-Inda-1-(o-diethylaminobenzyl)-cyclohexan
1-Inda-1-(o-dimethylaminobenzyl)-cyclooctan
2,5-Dimethyl-1-(3-dimethylaminopropyl)-2,5-diaza-1-alumina-cyclopentan

2,5-Dimethyl-1-(3-dimethylaminopropyl)-2,5-diaza-1-alumina-cyclohexan
2,5-Dimethyl-1-(3-dimethylaminopropyl)-2,5-diaza-1-alumina-cycloheptan
2,5-Dimethyl-1-(3-dimethylaminopropyl)-2,5-diaza-1-alumina-cyclopentan
2,5-Dimethyl-1-(3-diethylaminopropyl)-2,5-diaza-1-alumina-cyclohexan
2,5-Dimethyl-1-(3-diethylaminopropyl)-2,5-diaza-1-alumina-cycloheptan
2,5-Diethyl-1-(3-dimethylaminopropyl)-2,5-diaza-1-alumina-cyclohexan
2,5-Diethyl-1-(3-dimethylaminopropyl)-2,5-diaza-1-alumina-cyclopentan
2,5-Diethyl-1-(4-dimethylaminobutyl)-2,5-diaza-1-alumina-cyclohexan
2,5-Diethyl-1-(4-dimethylaminobutyl)-2,5-diaza-1-alumina-cyclopentan
2,5-Diethyl-1-(4-dimethylaminobutyl)-2,5-diaza-1-alumina-cycloheptan
2-Ethyl-5-propyl-1-(4-dimethylaminobutyl)-2,5-diaza-1-alumina-cyclohexan
2-Ethyl-5-propyl-1-(4-dimethylaminobutyl)-2,5-diaza-1-alumina-cyclopentan
2-Ethyl-5-propyl-1-(4-dimethylaminobutyl)-2,5-diaza-1-alumina-cycloheptan
2,5-Dimethyl-1-(3-dimethylaminopropyl)-2,5-diaza-1-galla-cyclopentan, Kp. 220 °C/10 Pa (0,1 mbar)
2,5-Dimethyl-1-(3-dimethylaminopropyl)-2,5-diaza-1-galla-cyclohexan
2,5-Dimethyl-1-(3-dimethylaminopropyl)-2,5-diaza-1-galla-cycloheptan
2,5-Dimethyl-1-(3-diethylaminopropyl)-2,5-diaza-1-galla-cyclopentan
2,5-Dimethyl-1-(3-diethylaminopropyl)-2,5-diaza-1-galla-cyclohexan
2,5-Dimethyl-1-(3-diethylaminopropyl)-2,5-diaza-1-galla-cycloheptan
2,5-Diethyl-1-(3-dimethylaminopropyl)-2,5-diaza-1-galla-cyclohexan
2,5-Diethyl-1-(3-dimethylaminopropyl)-2,5-diaza-1-galla-cyclopentan
2,5-Diethyl-1-(3-dimethylaminopropyl)-2,5-diaza-1-galla-cycloheptan
2,5-Diethyl-1-(4-dimethylaminobutyl)-2,5-diaza-1-galla-cyclohexan
2,5-Diethyl-1-(4-dimethylaminobutyl)-2,5-diaza-1-galla-cyclopentan
2,5-Diethyl-1-(4-dimethylaminobutyl)-2,5-diaza-1-galla-cycloheptan
2-Ethyl-5-propyl-1-(4-dimethylaminobutyl)-2,5-diaza-1-galla-cyclohexan
2-Ethyl-5-propyl-1-(4-dimethylaminobutyl)-2,5-diaza-1-galla-cyclopentan
2-Ethyl-5-propyl-1-(4-dimethylaminobutyl)-2,5-diaza-1-galla-cycloheptan
2,5-Dimethyl-1-(3-dimethylaminopropyl)-2,5-diaza-1-inda-cyclopentan
2,5-Dimethyl-1-(3-dimethylaminopropyl)-2,5-diaza-1-inda-cyclohexan
2,5-Dimethyl-1-(3-dimethylaminopropyl)-2,5-diaza-1-inda-cycloheptan
2,5-Dimethyl-1-(3-diethylaminopropyl)-2,5-diaza-1-inda-cyclopentan
2,5-Dimethyl-1-(3-diethylaminopropyl)-2,5-diaza-1-inda-cyclohexan
2,5-Dimethyl-1-(3-diethylaminopropyl)-2,5-diaza-1-inda-cycloheptan
2,5-Diethyl-1-(3-dimethylaminopropyl)-2,5-diaza-1-inda-cyclohexan
2,5-Diethyl-1-(3-dimethylaminopropyl)-2,5-diaza-1-inda-cyclopentan
2,5-Diethyl-1-(3-dimethylaminopropyl)-2,5-diaza-1-inda-cycloheptan
2,5-Diethyl-1-(3-dimethylaminobutyl)-2,5-diaza-1-inda-cyclohexan
2,5-Diethyl-1-(3-dimethylaminobutyl)-2,5-diaza-1-inda-cyclopentan
2,5-Diethyl-1-(3-dimethylaminobutyl)-2,5-diaza-1-inda-cycloheptan
2-Ethyl-5-propyl-1-(4-dimethylaminobutyl)-2,5-diaza-1-inda-cyclohexan
2-Ethyl-5-propyl-1-(4-dimethylaminobutyl)-2,5-diaza-1-inda-cyclopentan
2-Ethyl-5-propyl-1-(4-dimethylaminobutyl)-2,5-diaza-1-inda-cycloheptan

Typische Beispiele von Verbindungen der Formel II sind:

5-Methyl-1-galla-5-aza-cyclooctan, Fp. -22 °C
1,5-Dimethyl-1-galla-5-aza-cyclooctan, Kp. 83 °C/1.2 kPa (12 mbar)
1,5-Diethyl-1-galla-5-aza-cyclooctan
1,5-Dipropyl-1-galla-5-aza-cyclooctan
1,5-Dimethyl-1-alumina-5-aza-cyclooctan, Kp. 76 °C/0.4 kPa (4 mbar)
1,5-Diethyl-1-alumina-5-aza-cyclooctan, Kp. 71 °C/60 Pa (0,6 mbar)
1,5-Diisopropyl-1-alumina-5-aza-cyclooctan
1,5-Dibutyl-1-alumina-5-aza-cyclooctan
1-Methyl-5-ethyl-1-galla-5-aza-cyclooctan
1-Ethyl-5-methyl-1-alumina-5-aza-cyclooctan, Kp. 71 °C/60 Pa (0,6 mbar)
1,6-Dimethyl-1-galla-6-aza-cyclodecan
1,6-Dimethyl-1-alumina-6-aza-cyclodecan

1,6-Diethyl-1-galla-6-aza-cyclodecan
1,4-Dimethyl-1-galla-4-aza-cyclohexan
1,6-Diethyl-1-alumina-6-aza-cyclodecan
1-Galla-5-aza-bicyclo(3.3.3)-undecan, Fp. 54 °C
1-Galla-4-aza-bicyclo(2.2.2)-octan
1-Alumina-5-aza-bicyclo(3.3.3)-undecan, Kp. 80 °C/40 Pa (0,4 mbar)
1-Alumina-4-aza-bioyclo(2.2.2)-octan
1-Galla-6-aza-bicyclo(4.4.4)-tetradecan
1-Alumina-6-aza-bicyclo(4.4.4)-tetradecan
1,5-Dimethyl-1-inda-5-aza-cyclooctan, Kp. 38 °C/5 Pa (0,05 mbar)
1,5-Diethyl-1-inda-5-aza-cyclooctan
1,5-Dipropyl-1-inda-5-aza-cyclooctan
1,5-Diisopropyl-1-inda-5-aza-cyclooctan
1,5-Dibutyl-1-inda-5-aza-cyclooctan
1-Methyl-5-ethyl-1-inda-5-aza-cyclooctan
1-Ethyl-5-propyl-1-inda-5-aza-cyclooctan
1,6-Dimethyl-1-inda-6-aza-cyclodecan
1,6-Diethyl-1-inda-6-aza-cyolodecan
1,4-Dimethyl-1-inda-4-aza-cyclohexan
1-Inda-5-aza-bicyclo(3.3.3)-undecan
1-Inda-4-aza-bicyclo(2.2.2)-octan
1-Methyl-5-cyclohexyl-1-inda-5-aza-cyclooctan
1-Methyl-5-phenyl-1-inda-5-aza-cyclooctan
1-Inda-6-aza-bicyclo(4.4.4)-tetradecan
1,6-Dimethyl-1-galla-6-aza-cyclodecan
1,6-Diethyl-1-galla-6-aza-cyclodecan
1,6-Dipropyl-1-galla-6-aza-cyclodecan
1,6-Diisopropyl-1-galla-6-aza-cyclodecan
1,6-Dibutyl-1-galla-6-aza-cyclodecan
1,6-Di-tert.butyl-1-galla-6-aza-cyclodecan
1,6-Di-isobutyl-1-galla-6-aza-cyclodecan
1,4-Dimethyl-1-galla-4-aza-cyclohexan
1,4-Diethyl-1-galla-4-aza-cyclohexan
1,4-Dipropyl-1-galla-4-aza-cyclohexan
1,4-Diisopropyl-1-galla-4-aza-cyclohexan
1,4-Dibutyl-1-galla-4-aza-cyclohexan
1,4-Di-isobutyl-1-galla-4-aza-cyclohexan
1,4-Di-tert.butyl-1-galla-4-aza-cyclohexan
1-Methyl-5-ethyl-1-galla-5-aza-cyclooctan
1-Methyl-5-propyl-1-galla-5-aza-cyclooctan
1-Propyl-5-methyl-1-galla-5-aza-cyclooctan, Kp. 86 °C/1 Pa (0,01 mbar)
1-Ethyl-5-methyl-1-galla-5-aza-cyclooctan, Kp. 64 °C/100 Pa (1 mbar)
1-Ethyl-6-propyl-1-galla-6-aza-cyclodecan
1-Propyl-6-butyl-1-galla-6-aza-cyclodecan
1-Methyl-6-ethyl-1-galla-6-aza-cyclodecan
1-Methyl-4-ethyl-1-galla-4-aza-cyclohexan
1-Propyl-4-methyl-1-galla-4-aza-cyclohexan
1-Ethyl-4-butyl-1-galla-4-aza-cyclohexan

Bevorzugt sind aluminiumorganische Verbindungen der Formeln I und II.

Besonders bevorzugt sind die Verbindungen 1-Alumina-1-(3-dimethylaminopropyl)-cyclohexan und 1,5-Dimethyl-1-alumina-5-aza-cyclooctan.

Die meisten zyklischen metallorganischen Verbindungen der Formeln I und II sind als solche an sich bereits bekannt. So sind Verbindungen der Formel I in DE 3817090 sowie in DE 4009394 und Verbindungen der Formel II in DE 3726485 erstmals beschrieben. Den genannten Dokumenten ist bereits zu entnehmen, daß die Verbindungen gegenüber Luft und Feuchtigkeit stabil sind.

Jedoch werden sie nur zur Verwendung für die Herstellung dünner Metall- bzw. Verbindungshalbleiterschichten durch Gasphasenabscheidung vorgeschlagen. Ein Hinweis auf die Eignung dieser Verbindungen als aktivierende Komponenten in Koordinations-Katalysatorsystemen für die Olefin-Polymerisation oder Metathese ist diesen Dokumenten nicht zu entnehmen.

Die Verbindungen der Formeln I und II werden nach an sich bekannten Methoden hergestellt, wie sie in der Literatur (z.B. G. Bähr, P. Burba, Methoden der Organischen Chemie, Bd. XIII/4, Georg Thieme Verlag, Stuttgart (1970)) beschrieben sind, und zwar unter Reaktionsbedingungen, die für die genannten Umsetzungen bekannt und geeignet sind. Dabei kann man auch von an sich bekannten, hier nicht erwähnten Varianten Gebrauch machen.

So können diese Verbindungen etwa hergestellt werden, indem man Metallalkylchloride mit einem Alkalimetallorganyl der entsprechenden Lewisbase oder einer Grignard-Verbindung in einem inerten Lösungsmittel umsetzt.

Nähere Details zur Synthese dieser Verbindungen können den vorgenannten Patentdokumenten oder Chem. Ber. 124, 1113-1119 (1991) entnommen werden.

Koordinations-Katalysatorsysteme, die die erfindungsgemäßen zyklischen metallorganischen Verbindungen der Formeln I und II enthalten, eignen sich im besonderen für die Polymerisation von 1-Alkenen und 1-Alkinen, wobei sowohl Homopolymere aus einheitlichen Monomeren als auch Copolymere aus Monomergemischen erhalten werden können. Weiterhin eignen sie sich für die Metathese von 1-Alkenen und 1-Alkinen sowie für die ringöffnende Metathese von zyklischen Alkenen.

Bei dem erfindungsgemäßen Einsatz der Verbindungen der Formeln I und II als aktivierende Komponenten in Koordinations-Katalysatorsystemen hat sich überaschend herausgestellt, daß die Katalysatorsysteme hierdurch weniger empfindlich gegenüber (Luft-)Sauerstoff und Feuchtigkeit reagieren, so daß nicht derartig strikte Schutzmaßnahmen getroffen werden müssen, wie sie bei mit üblichen Aluminiumalkylen aktivierten Koordinations-Katalysatoren erforderlich sind. Dieser Befund ist besonders überaschend und nicht vorhersehbar, zumal der Einsatz strukturell ähnlicher, jedoch nichtzyklischer aluminiumorganischer Verbindungen, wie beispielsweise (3-Diethylaminopropyl)-di-isobutylaluminium, in bestimmten Koordinations-Katalysatorsystemen bereits bekannt ist. In US 3,154,528 werden mit derartigen nichtzyklischen Verbindungen aktivierte Koordinations-Katalysatorsysteme auf Basis von Vanadiumtetrachlorid offenbart, die jedoch als genauso luft- und feuchtigkeitsempfindlich beschrieben werden, wie übliche Koordinations-Katalysatorsysteme. JP 60-240706, JP 61-007305, JP 61-252205, JP 62-100505 und JP 62-138506 offenbaren Koordinations-Katalysatorsysteme auf Basis von Titanverbindungen, die ebenfalls derartige nichtzyklische aluminiumorganische Verbindungen sowie noch weitere, als Elektronen-Donatoren fungierende Verbindungen enthalten, die aber zunächst mit üblichen Aluminiumalkylen aktiviert werden. Aufgrund der Anwesenheit letzterer sind diese Katalysatorsysteme ebenfalls äußerst empfindlich und bedürfen der üblichen strikten Schutzmaßnahmen.

Eigene Untersuchungen mit diesen bekannten nichtzyklischen aluminiumorganischen Verbindungen in Ziegler-Natta- und Phillips-Katalysatorsystemen bestätigen auch, daß diese unbedingt die Aktivierung mit üblichen Aluminiumalkylen benötigen, um überhaupt ein Olefinpolymer oder zumindest brauchbare Produktausbeuten zu erhalten.

Bei den erfindungsgemäßen Koordinations-Katalysatorsystemen ist eine zusätzliche Aktivierung durch Aluminiumalkyle oder sonstige Aktivatoren nicht erforderlich.

Bei den erfindungsgemäßen Koordinations-Katalysatorsystemen hat ein moderater Zutritt von Luft, Sauerstoff oder Feuchtigkeit keine Zerstörung oder drastische Aktivitätsverringerung zur Folge. Entsprechende Koordinations-Katalysatorsysteme auf Basis von trägergebundenem Chrom glühen beispielsweise an Luft nicht auf, sondern sind weiterhin unverändert gut einsetzbar. Dies hat die sehr vorteilhafte Folge, daß die Handhabung der erfindungsgemäßen Koordinations-Katalysatorsysteme bei Herstellung, Lagerung und Gebrauch wesentlich unproblematischer ist. Auf den aufwendigen Ausschluß bereits von Spuren von Luft, Sauerstoff und Feuchtigkeit in den bei der Polymerisation eingesetzten Lösungsmitteln, Monomeren und Schutzgasen kann daher verzichtet werden.

Darüberhinaus bewirken die erfindungsgemäß in Koordinations-Katalysatorsystemen einzusetzenden Verbindungen der Formeln I und II weitere vorteilhafte Ergebnisse beim bestimmungsgemäßen Einsatz in Polymerisationsverfahren und Metathesereaktionen. So zeigen die entsprechenden erfindungsgemäßen Koordinations-Katalysatorsysteme generell eine äußerst hohe Aktivität. Dies hat zur Folge, daß mit der eingesetzten Menge an Katalysator mehr Produkt gebildet wird bzw. die erforderliche Menge an Katalysator entsprechend reduziert werden kann. Vorteilhafte Folgen hiervon sind, daß entsprechend weniger an Katalysator vom Produkt abgetrennt werden muß bzw. Produkte mit geringerem Restgehalt an Katalysator erhalten und nicht zuletzt auch die Kosten aufgrund geringeren Katalysatorverbrauchs reduziert werden. Naturgemäß sind hierbei zahlreiche weitere Faktoren mit beeinflußend, wie qualitative und quantitative Zusammensetzung des Katalysatorsystems, Natur der Monomeren bzw. die Zusammensetzung bei der Copolymerisation von Monomergemischen, Reaktionsbedingungen und Fahrweise bei der Polymerisation. Der Fachmann kann jedoch ohne weiteres mit Hilfe von Routineversuchen das für seine Zwecke geeignetste Katalysatorsystem ermitteln und optimieren. So zeigte beispielsweise ein Ziegler-Natta-Katalysatorsystem auf Basis von $Ti/MgCl_2$, das mit der Verbindung 1-Alumina-1-(3-dimethylaminopropyl)-cyclohexan als Cokatalysator aktiviert wurde, bei der Polymerisation von Ethylen im technischenMaßstab dann ein Optimum hinsichtlich gleichbleibender Aktivität und Produktausbeute, wenn in dem Katalysatorsystem das Molverhältnis Ti zu Al im Bereich 1:40-50 lag.

Weiter zeigt sich für die erfindungsgemäßen Koordinations-Katalysatorsysteme bei der Olefin-Polymerisation eine ausgeprägte Spezifität in Richtung auf hohe Molmassen und engere Molmassenverteilungen. Auch diese Befunde sind

abhängig von Faktoren, wie Zusammensetzung des Katalysatorsystems, Natur der Monomere und angewendete Verfahrensbedingungen, können aber ohne weiteres für den jeweiligen Anwendungsfall optimiert werden. So fanden sich beispielsweise bei der Polymerisation von 1-Octen sowie 1-Decen mit Phillips-Katalysatorsystemen auf Basis von $Cr/SiO_2$, die mit 1-Alumina-1-(3-dimethylaminopropyl)-cyclohexan als Cokatalysator aktiviert wurden, bei Variation des Molverhältnisses Cr zu Al im Bereich 1:0,5 bis 1:4 durchwegs hohe Produktausbeuten und hohe Molmassen und für 1-Octen ein Optimum hinsichtlich enger Molmassenverteilung bei einem Verhältnis Cr zu Al von 1:1.

Als weiterer vorteilhafter Befund hat sich herausgestellt, daß mit Verbindungen der Formeln I und II aktivierte Phillips-Katalysatoren nicht, wie sonst üblich, mit dem Polymer verkleben und an den Reaktionsapparaturen haften bleiben, wodurch die Entfernung und Abtrennung erheblich erleichtert ist.

Metathese-Katalysatoren auf Basis von $Mo/SiO_2$, aktiviert mit 1-Alumina-1-(3-dimethylaminopropyl)-cyclohexan, zeigen eine überaschend hohe Produktspezifität. So entstand beispielsweise bei der Metathese von 1-Octen mit dem genannten Katalysatorsystem ausschließlich das gewünschte $C_{14}$-Alken, während ein mit Triisobutylaluminium aktiviertes Katalysatorsystem dieser Art neben dem gewünschten Produkt einen hohen Nebenproduktanteil in Form eines $C_2$- bis $C_{20}$-Alkengemisches lieferte.

Bei der Polymerisation von 1-Alkenen können im gegebenen Fall die erfindungsgemäßen Katalysatoren ausgeprägt stereoselektiv in Richtung auf isotaktische Polymere wirken. Bei der Metathese bzw. ringöffnenden Metathesepolymerisation können bestimmte Stereoisomere bzw. Produkte mit bestimmter Konfiguration bevorzugt entstehen. Diese Stereoselektivität läßt sich darüberhinaus durch gezielte Strukturierungen der Verbindungen der Formel I und II, insbesondere an deren zyklischen Strukturelementen beeinflußen. Ist etwa in Formel I für den Fall, daß $X^1$ und/oder $X^2$ $CHR^1$ bedeuten, der Rest $R^1$ nicht Wasserstoff, so weist das zyklische Strukturelement ein asymmetrisches C-Atom und somit ein Chiralitätszentrum auf, durch das bei Polymerisation oder Metathese von 1-Alkenen eine stereoselektive Induktion bewirkt werden kann. Eine derartige stereoselekive oder gegebenenfalls enantioselektive Induktion durch ein entsprechendes Katalysatorsystem ist vorzugsweise dann zu erwarten, wenn die Verbindungen der Formel I in enantiomeren- oder diastereomerenreiner Form eingesetzt werden.

Die Ziegler-Natta und die Phillips-Polymerisation von Ethylen und Propylen gelingt mit den erfindungsgemäßen Koordinations-Katalysatorsystemen problemlos bereits unter milden Bedingungen, wie z.B. bei ca. 70 °C und unter Druck von 8-10 bar. Im Vergleich zu einer Aktivierung mit üblichen Aluminiumalkylen, wie beispielsweise Triisobutylaluminium, werden hier überwiegend höhere Produktausbeuten und vor allem deutlich höhere Molmassen erzielt. Olefinpolymere mit besonders hoher Mol-masse, etwa über $10^6$, wie mit den erfindungsgemäßen Katalysatorsystemen ohne weiteres im Labormaßstab und ohne technische Optimierung erhältlich, sind von großer technischer Bedeutung.

Koordinations-Katalysatorsysteme vom "Ziegler-Natta"- und vom "Phillips"-Typ, die mit den erfindungsgemäßen Verbindungen der Formeln I und II als Cokatalysatoren aktiviert werden, zeigen darüberhinaus die überraschende Eigenschaft, daß mit ihnen auch polare olefinisch ungesättigte Monomere polymerisiert werden können. Bekanntlich lassen sich mit konventionellen Katalysatorsystemen im wesentlichen nur 1-Alkene und 1-Alkine polymerisieren. Styrol, beispielsweise, läßt sich kaum noch und noch polarere Monomere wie Vinylverbindungen und Acrylsäurederivate lassen sich gar nicht der Koordinationspolymerisation unterziehen. Vermutlich liegt dies daran, daß diese polaren Verbindungen die Metallatome von Primär- und/oder Cokatalysator durch eine starke koordinative Bindung desaktivieren. Durch die intramolekulare koordinative Absättigung bei den Verbindungen der Formeln I und II ist dies jedoch nicht mehr möglich, was die bemerkenswerte Aktivität der erfindungsgemäßen Katalysatorsysteme gegenüber polaren Monomeren erkärt.

Der erfindungsgemäße Einsatz der Verbindungen der Formeln I und II als aktivierende Komponenten in Koordinations-Katalysatorsystemen erfolgt völlig analog und im Ersatz zu den bisher üblichen Metallorganylen und insbesondere den äußerst empfindlichen und gefährlichen Aluminiumalkylen. Aufgrund der gesteigerten Aktivität kann der Anteil an metallorganischen Verbindung im Katalysatorsystem wie auch die Menge an Katalysator bei der Umsetzung reduziert werden. Die Herstellung und Anwendung der Katalysatoren erfolgt in an sich bekannter Weise wie sie für das jeweilige System und den jeweiligen Einsatz üblich ist. In der Regel wird bei der Olefin-Polymerisation und bei der Metathese mit heterogener Katalyse im Suspensionsverfahren gearbeitet. Hierzu wird zunächst aus der katalytische aktiven Übergangsmetallverbindung und einem feinteiligen Trägermaterial der trägergebundene Präkatalysator hergestellt, dieser erforderlichenfalls in üblicher Weise aktiviert bzw. voraktiviert und dann in einem Lösungsmittel, z.B. in einem Alkankohlenwasserstoff wie Pentan oder Hexan, suspendiert. Die Zugabe des Cokatalysators erfolgt wie sonst auch üblich unmittelbar vor der Umsetzung der Monomere oder "in situ" in Anwesenheit derselben. Die Steuerung der Reaktion sowie die Gewinnung und Aufarbeitung der Reaktionsprodukte erfolgt ebenfalls in völlig analoger Weise. Wie schon erwähnt sind aufgrund der Stabilität der Verbindungen der Formeln I und II und der geringeren Empfindlichkeit der resultierenden Katalysatorsyteme alle diese Handlungen wesentlich problemloser und mit wesentlich weniger strikten Schutz- und Sicherheitsmaßnahmen durchführbar.

Durch die Erfindung werden somit neue Koordinations-Katalysatorsysteme mit vorteilhaften Eigenschaften sowie erheblich erweiterter Einsatzbreite zugänglich gemacht, die darüberhinaus auf die jeweiligen Anwendungsbedürfnisse hin maßgeschneidert werden können.

In den nachfolgenden Beispielen werden alle Handhabungen, sofern nicht anderes angegeben, unter Schutzgasatmosphäre ($N_2$, Ar) und unter Ausschluß von Feuchtigkeit vorgenommen.

Beispiel 1:

0,25 mol Magnesiumspäne, durch lod aktiviert, werden in 100 ml Diethylether vorgelegt. Nach Zugabe von 0,06 mol 1,5-Dibrompentan bei Raumtemperatur wird 3 Stunden unter Rückfluß erwärmt.

Die vom Magnesium abdekantierte Grignardlösung und 0,06 mol 3-Dimethylaminopropylaluminiumdichlorid, in 150 ml Ether gelöst, werden synchron unter kräftigem Rühren zur Reaktion zusammengeführt.

Anschließend wird das Reaktionsgemisch bei Raumtemperatur gerührt. Die flüchtigen Bestandteile werden bei einer Badtemperatur bis zu 180° und einem Druck von 10-2 mbar abdestilliert und nochmals fraktioniert destilliert.

Man erhält 1-Alumina-1-(3-dimethylaminopropyl)-cyclohexan als wasserklare, luftstabile Flüssigkeit mit Siedepunkt 98°/0,6 mbar.

Beispiel 2:

2,9 g (119 mmol) Magnesiumgrieß werden in 100 ml THF vorgelegt und unter Rückfluß erwärmt. Es werden 10 g (54 mmol) Methylamino-bis-(3,3'-propylchlorid), gelöst in 40 ml THF, zugegeben. Anschließend erwärmt man noch 2 Stunden unter Rückfluß.

Zu der Grignard-Lösung werden bei Raumtemperatur 5,7 g (50 mmol) Methylaluminiumdichlorid in 20 ml THF gegeben. Man rührt 24 Stunden bei Raumtemperatur und erhitzt dann 3 Stunden unter Rückfluß. Man dekantiert von ausgefallenem $MgCl_2$ und erhält nach Abziehen des Lösungsmittels 1,5-Dimethyl-1-alumina-5-aza-cyclooctan durch Vakuumdestillation als klare, stabile Flüssigkeit mit Siedepunkt 71°/0,6 mbar.

Beispiel 3:

3.6 g (148 mmol) Magnesiumgrieß werden in 100 ml THF vorgelegt und unter Rückfluß erwärmt. Es werden 12 g (49 mmol) 3-Chlor-N,N-bis-(3-chlorpropyl)-1propanamin in 40 ml THF zugegeben, und man erhitzt noch 2 Stunden.

Zu der Grignard-Lösung werden bei Raumtemperatur 6,6 g (47 mmol) Aluminumtrichlorid in 20 ml THF gegeben. Man rührt 24 Stunden bei Raumtemperatur und erhitzt dann 4 Stunden unter Rückfluß. Man erhält 1-Alumina-5-aza-bicyclo(3.3.3)undecan nach Abdestillieren des Lösungsmittels und Reinigung durch Vakuumdestillation als klare, stabile Flüssigkeit mit Siedepunkt 80°/40 Pa (0,4 mbar).

Beispiel 4: Trägergebundener Cr(VI)-Präkatalysator

1000 g Silicagel (Partikelgröße 200-500 mm) werden mit 3000 ml dest. $H_2O$ 45 Min. ausgekocht, mit heißem $H_2O$ dreimal gewaschen und 15 Std. bei 115 °C/75 mbar getrocknet. Anschließend wird in einer Lösung von 46 g $CrO_3$ in 200 ml Wasser aufgeschlämmt, 30 Min. gerührt, abfiltriert und bei 70 °C/75 mbar 12 Std., danach 2 Std. bei 115°/75 mbar getrocknet. Das Produkt wird nun im Sauerstoffstrom (Wirbelbett oder Drehrohr) im Laufe von 6 Std. auf 800 °C aufgeheizt und 1 Std. bei dieser Temperatur belassen. Nach Abkühlen auf 350 °C wird der Sauerstoff durch Argon ersetzt. Der Katalysator enthält ca. 1 % Cr(VI).

Beispiel 5: Trägergebundener Cr(II)-Präkatalysator

Man verfährt zunächst wie in Beispiel 5. Argon wird nun aber durch CO verdrängt; die Reduktion erfolgt im CO-Strom bei 350 °C/60 Min. Abschließend wird das CO wieder durch Argon ersetzt und auf Raumtemperatur abgekühlt. Der Katalysator enthält ca. 0,84 % Cr(II).

Beispiel 6: Trägergebundener Mo(VI)-Präkatalysator

1,67 mmol $MoO_2$-Acetylacetonat-Komplex werden in 30 ml $CH_2Cl_2$ gelöst und auf 9 g Silicagel (Partikelgröße 200-500 mm) aufgezogen. Anschließend wird mit $CH_2Cl_2$ gewaschen und bei -10 °C im Hochvakuum getrocknet.

Beispiel 7: Polymerisation von 1-Octen an Cr(VI)

In Parallelansätzen wird der Präkatalysator gemäß Beispiel 4 in n-Pentan suspendiert und mit unterschiedlichen Mengen der Verbindung nach Beispiel 1 als Cokatalysator versetzt. Anschließend wird 1-Octen im Verhältnis Cr: 1-Octen wie 1:100 zugegeben und 24 Stunden bei 20 °C geschüttelt. Der Katalysator wird abfiltriert und mit n-Pentan

gewaschen. Die Pentaneluate werden eingedampft. Das Polymerprodukt wird durch IR- und $^{13}C$-NMR-Spektroskopie sowie durch Gelpermeationschromatographie (Polystyrol-Standard) analysiert. Tabelle 1 zeigt die Ergebnisse.

Tabelle 1

| Cr : Al | Ausbeute in % | Mw | D = Mw/Mn |
|---|---|---|---|
| 1 : 0,5 | 55 | 10900 | 8,45 |
| 1 : 1 | 86 | 21900 | 6,35 |
| 1 : 1,5 | 67 | 15650 | 9,1 |
| 1 : 2 | 43 | 14900 | 8,4 |

Es wurden durchweg bei guten Ausbeuten hohe Molmassen (Mw) bei engen Molmassenverteilungen (D = Mw/Mn) erhalten. Der Katalysator mit Cr:Al wie 1:1 lieferte optimale Ergebnisse.

Beispiel 8: Polymerisation von 1-Octen an Cr(II)

Mit dem Präkatalysator gemäß Beispiel 5 wird wie in Beispiel 7 vorgegangen. Tabelle 2 zeigt die Ergebnisse.

Tabelle 2

| Cr : Al | Ausbeute in % | Mw | D = Mw/Mn |
|---|---|---|---|
| 1 : 1 | 91 | 37900 | 2,1 |
| 1 : 2 | 77 | 62800 | 11,0 |
| 1 : 4 | 68 | 78700 | 7,9 |
| Vergleichsversuch mit Triethylaluminium als Cokatalysator | | | |
| 1 : 2,7 | 52 | 31800 | breit/bimodal |

Beispiel 9: Polymerisation von 1-Decen an Cr(II)

Mit 1-Decen wird wie in Beispiel 8 vorgegangen. Tabelle 3 zeigt die Ergebnisse.

Tabelle 3

| Cr : Al | Ausbeute in % | Mw | D = Mw/Mn |
|---|---|---|---|
| 1 : 2 | 83 | 47200 | 9,8 |
| 1 : 3 | 97 | 77700 | 11,6 |
| 1 : 4 | 85 | 53200 | 14,5 |

Beispiel 10: Metathese von 1-Octen an Mo(VI)

In Parallelansätzen werden jeweils 300 mg des Präkatalysators gemäß Beispiel 6 (entsprechend 0,05 mmol Mo) mit 1-Octen im Verhältnis Mo: 1-Octen wie 1:2500 versetzt und unterschiedliche Mengen der Verbindung nach Beispiel 1 als Cokatalysator zugesetzt. Man läßt 24 Stunden bei 122 °C reagieren. Der Umsatz an 1-Octen und das entstandene Tetradecen ($C_{14}$) sowie eventuelle Nebenprodukte werden gaschromatographisch bestimmt.

In einem analogen Vergleichsversuch wird Triisobutylaluminium (TIBA) als Cokatalysator eingesetzt. Tabelle 4 zeigt die Ergebnisse.

Tabelle 4

| Mo : Al | Ausbeute in % Produkt $C_{14}$ | Ausbeute in % Nebenprodukte $C_2$-$C_{20}$ |
|---|---|---|
| 1 : 2 | 14,8 | - |
| 1 : 4 | 19,4 | - |
| 1 : 10 | 4,8 | - |
| Vergleichsversuch mit TIBA | | |
| 1 : 4 | 12,3 | 16,2 |

Beispiel 11: Polymerisation von 1-Octen an Cr(II) mit und ohne Sauerstoffzutritt

Es wird wie in Beispiel 8 verfahren. In einem Parallelansatz wird vor der Zugabe des Alkens 5 ml $O_2$-Gas mittels Spritze injiziert. Tabelle 5 zeigt die Ergebnisse.

Tabelle 5

| Cr : Al | Ausbeute in % | Mw | D = Mw/Mn |
|---|---|---|---|
| Zusatz von $O_2$ | | | |
| 1 : 3 | 73 | 76500 | 13,4 |
| kein Zusatz von $O_2$ | | | |
| 1 : 3 | 87 | 78700 | 7,9 |

Das Ergebnis belegt, daß der Zusatz von $O_2$ keinen wesentlichen Einfluß auf die Aktivität des Katalysatorsystems ausübt.

Beispiel 12: Polymerisation von Ethylen an Cr(VI)

In Parallelansätzen werden jeweils 200 mg an Präkatalysator gemäß Beispiel 4 im Autoklaven in n-Heptan suspendiert. Hierzu werden unterschiedliche Mengen an Verbindung nach Beispiel 1 bzw. zum Vergleich an Cokatalysatoren nach dem Stand der Technik zugegeben und dann bei einer Temperatur von 70 °C Ethylen unter einem Druck von 1 MPa (10 bar) zugeführt. Nach 24 Stunden wird wie üblich aufgearbeitet. Tabelle 6 zeigt die Ergebnisse.

Tabelle 6

| Cr : Al | Ausbeute g Polymer/g Cr • h | Fp °C | Mw | Erscheinungsform |
|---|---|---|---|---|
| 1 : 0 | 0 | - | - | kein Polymer |
| 1 : 1 | 1700 | 189-192 | $1,02 \cdot 10^6$ | weißes, feinkörniges Polymer |
| 1 : 5 | 1250 | 175-200 | $1,03 \cdot 10^6$ | weißes, feinkörniges Polymer |
| 1 : 10 | 1300 | 188-194 | $1,2 \cdot 10^6$ | weißes, feinkörniges Polymer |
| 1 : 15 | 1400 | 191-195 | $1,1 \cdot 10^6$ | weißes, feinkörniges Polymer |
| Vergleichsversuche mit TIBA | | | | |
| 1 : 1 | 1200 | 185-188 | $8,5 \cdot 10^5$ | weißes, feinkörniges Polymer |
| 1 : 15 | 1500 | 174-177 | $5,9 \cdot 10^5$ | weißes, feinkörniges Polymer |
| Vergleichsversuch mit (2-Dimethylaminoethoxy)-dimethylaluminium | | | | |
| 1 : 25 | 0 | - | - | kein Polymer |

Der erfindungsgemäße Cokatalysator bewirkt vorzügliche Ausbeuten und gegenüber TIBA eine Erhöhung der Molmasse. Die nichtzykliche Verbindung bewirkt keine Polymerisation.

Bespiel 13: Ziegler-Natta-Polymerisation von Ethylen

In Parallelansätzen werden jeweils 50 mg $TiCl_4$ im Autoklaven in n-Heptan suspendiert und unterschiedliche Mengen an Verbindung nach Beispiel 1 bzw. zum Vergleich an Cokatalysatoren nach dem Stand der Technik zugegeben. Bei einer Temperatur von 70 °C wird dann Ethylen unter einem Druck von 0.8-1 MPa (8-10 bar) zugeführt und die Reaktion nach einer Stunde abgebrochen. Tabelle 7 zeigt die Ergebnisse

Tabelle 7

| Ti : Al | Ausbeute g Polymer/g Ti • h | Fp °C | Mw | Erscheinungsform |
|---|---|---|---|---|
| 1 : 3 | 14700 | 175-210 | $7,9 \cdot 10^6$ | feinkörnig weiß |
| 1 : 5 | 15400 | 190 (Zersetzung) | $2 \cdot 10^6$ | feinkörnig weiß |
| Vergleichsversuch mit TIBA | | | | |
| 1 : 3 | 18600 | 196-205 | $3,4 \cdot 10^5$ | grobkörnig grau |
| Vergleichsversuch mit (3-Dimethylaminopropyl)-diisopropylaluminium | | | | |
| 1 : 5 | 0 | - | - | kein Polymer |
| Vergleichsversuch mit (2-Dimethylaminoethoxy)-dimethylaluminium | | | | |
| 1 : 5 | - | - | - | Spuren an Polymer |

Der erfindungsgemäße Cokatalysator bewirkt vorzügliche Ausbeuten und gegenüber TIBA eine Erhöhung der Molmasse. Die nichtzyklischen Verbindungen bewirken keine bzw. eine spurenweise Polymerisation.

Beispiel 14: Technische Ziegler-Natta-Polymerisation von Ethylen

Die Polymerisation von Ethylen erfolgt im Suspensionsverfahren in n-Heptan bei einem konstanten Ethylen-Partialdruck von 0,1 MPa (1 bar), einer Temperatur von 80 °C sowie einer Rührgeschwindigkeit von 1500 U/min in Anwesenheit eines technischen Ziegler-Natta-Katalysators auf Basis von Ti/$MgCl_2$ mit 1 % Ti-Gehalt und der Verbindung nach Beispiel 1 als Cokatalysator.

Bei einem Verhältnis Ti:Al von 1:40 - 1:50 weist das Katalysatorsystem eine konstante Aktivität von 30-50 kg Polyethylen/g Ti • h • bar $C_2H_4$ auf.

**Patentansprüche**

1. Verwendung von cyclischen metallorganischen Verbindungen der Formeln I oder II

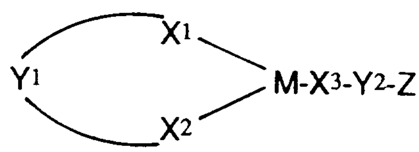

(I)

worin

| | |
|---|---|
| M | B, Al, Ga, In, |
| $X^1$, $X^2$, $X^3$ | jeweils unabhängig voneinander $CHR^1$, $NR^2$, O, S |
| $Y^1$, $Y^2$ | jeweils unabhängig voneinander $-(CH_2)_m-$,<br>$o-(CH_2)_p-C_6H_4-(CH_2)_q-$,<br>$o-(CH_2)_p-C_6H_6-(CH_2)_q-$,<br>$o-(CH_2)_p-C_6H_8-(CH_2)_q-$,<br>$o-(CH_2)_p-C_6H_{10}-(CH_2)_q-$,<br>$o-(CH_2)_p-C_5H_4-(CH_2)_q-$,<br>$o-(CH_2)_p-C_5H_6-(CH_2)_q-$,<br>$o-(CH_2)_p-C_5H_8-(CH_2)_q-$,<br>$-(CH_2)_p-CH=CH-(CH_2)_q-$, |
| Z | $NR^3R^4$, $PR^3R^4$, $OR^5$, $SR^5$ |
| $R^1$ | H, OH, Halogen, $C_{1-6}$-Alkyl oder $C_{1-6}$-Alkoxy, $C_{5-7}$-Cycloalkyl, Phenyl, |
| $R^2$, $R^3$, $R^4$, $R^5$ | jeweils unabhängig voneinander H oder $C_{1-6}$-Alkyl, $C_{5-7}$-Cycloalkyl, Phenyl, $R^3$ und $R^4$ zusammen auch eine $C_{4-6}$-Alkylenbrücke, |
| m | die Zahlen 1 bis 6, |
| p, q | jeweils unabhängig voneinander die Zahlen 0 bis 2 bedeuten, |

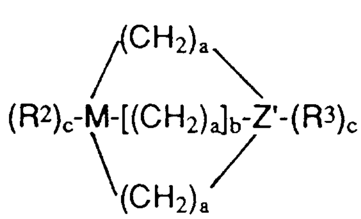

(II)

mit den vorstehenden Bedeutungen für M, $R^2$ und $R^3$ und worin

| | |
|---|---|
| Z' | N, P, |
| a | die Zahlen 2 bis 4, |

b, c die Zahlen 0 bis 1 mit $b + c = 1$ bedeuten,

als Komponenten in Koordinations-Katalysatorsystemen.

**2.** Verwendung nach Anspruch 1 in Koordinations-Katalysatorsystemen für die Koordinationspolymerisation und die Metathese von Alkenen und Alkinen.

**3.** Koordinations-Katalysatorsysteme auf Basis von Übergangsmetallverbindungen der IV. bis VIII. Nebengruppe und metallorganischen Verbindungen der III. Hauptgruppe des Periodensystems der Elemente, dadurch gekennzeichnet, daß sie mindestens eine Verbindung der Formel I oder II enthalten.

**4.** Verfahren zur Herstellung von Polymeren durch Koordinationspolymensation von Alkenen und/oder Alkinen, dadurch gekennzeichnet, daß ein Koordinations-Katalysatorsystem nach Anspruch 3 eingesetzt wird.

**5.** Verfahren zur Herstellung von ungesättigten Kohlenwasserstoffverbindungen durch katalysierte Metathesereaktion von Alkenen oder Alkinen, dadurch gekennzeichnet, daß als Metathesekatalysator ein Koordinations-Katalysatorsystem nach Anspruch 3 eingesetzt wird.

## Claims

**1.** Use of cyclic organometallic compounds of the formulae I or II

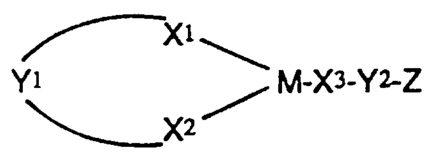

(I)

in which

| | |
|---|---|
| M | is B, Al, Ga, In, |
| $X^1$, $X^2$, $X^3$ | are, in each case independently of one another, $CHR^1$, $NR^2$, O, S, |
| $Y^1$, $Y^2$ | are, in each case independently of one another, $-(CH_2)_m-$, $o-(CH_2)_p-C_6H_4-(CH_2)_q-$, $o-(CH_2)_p-C_6H_6-(CH_2)_q-$, $o-(CH_2)_p-C_6H_8-(CH_2)_q-$, $o-(CH_2)_p-C_6H_{10}-(CH_2)_q-$, $o-(CH_2)_p-C_5H_4-(CH_2)_q-$, $o-(CH_2)_p-C_5H_6-(CH_2)_q-$, $o-(CH_2)_p-C_5H_8-(CH_2)_q-$, $-(CH_2)_p-CH=CH-(CH_2)_q-$, |
| Z | is $NR^3R^4$, $PR^3R^4$, $OR^5$, $SR^5$, |
| $R^1$ | is H, OH, halogen, $C_{1-6}$-alkyl or $C_{1-6}$-alkoxy, $C_{5-7}$-cycloalkyl, phenyl, |
| $R^2$, $R^3$, $R^4$, $R^5$ | are, in each case independently of one another, H or $C_{1-6}$-alkyl, $C_{5-7}$-cycloalkyl, phenyl, $R^3$ and $R^4$ together also a $C_{4-6}$-alkylene bridge, |
| m | is a number from 1 to 6, |
| p, q | are, in each case independently of one another, a number from 0 to 2, |

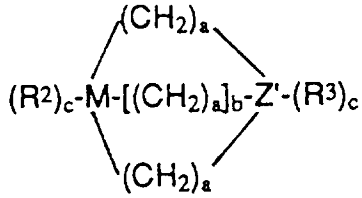

(II)

having M, $R^2$ and $R^3$ as defined above and in which

| | |
|---|---|
| Z' | is N, P, |
| a | is a number from 2 to 4, |
| b, c | are the numbers 0 or 1 with $b + c = 1$ , |

as components in coordination catalyst systems.

2. Use according to claim 1 in coordination catalyst systems for the coordination polymerization and metathesis of alkenes and alkynes.

3. Coordination catalyst systems based on transition metal compounds of subgroups IV to VIII and organometallic compounds of main group III of the Periodic Table of the Elements, characterized in that they contain at least one compound of the formula I or II.

4. Process for preparing polymers by coordination polymerization of alkenes and/or alkynes, characterized in that a coordination catalyst system according to claim 3 is used.

5. Process for preparing unsaturated hydrocarbon compounds by a catalyzed metathesis reaction of alkenes or alkynes, characterized in that the metathesis catalyst used is a coordination catalyst system according to claim 3.

**Revendications**

1. Utilisation de composés organométalliques cycliques de formule I ou II

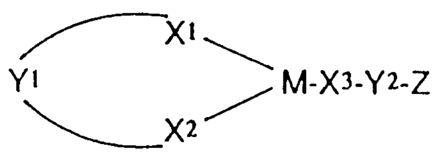

(I)

dans laquelle

| | |
|---|---|
| M | représente B, Al, Ga, In, |
| $X^1$, $X^2$, $X^3$ | représentent chacun, indépendamment les uns des autres, $CHR^1$, $NR^2$, O, S, |
| $Y^1$, $Y^2$ | représentent chacun, indépendamment l'un de l'autre,<br>$-(CH_2)_m$,<br>$o-(CH_2)_p-C_6H_4-(CH_2)_q-$,<br>$o-(CH_2)_p-C_6H_6-(CH_2)_q-$,<br>$o-(CH_2)_p-C_6H_8-(CH_2)_q-$,<br>$o-(CH_2)_p-C_6H_{10}-(CH_2)_q-$,<br>$o-(CH_2)_p-C_5H_4-(CH_2)_q-$,<br>$o-(CH_2)_p-C_5H_6-(CH_2)_q-$,<br>$o-(CH_2)_p-C_5H_8-(CH_2)_q-$,<br>$-(CH_2)_p-CH=CH-(CH_2)_q-$, |
| Z | représente $NR^3R^4$, $PR^3R^4$, $OR^5$, $SR^5$, |
| $R^1$ | représente H, OH, un atome d'halogène, un groupe alkyle en $C_{1-6}$ ou alcoxy en $C_{1-6}$, cycloalkyle en $C_{5-7}$, phényle, |
| $R^2$, $R^3$, $R^4$, $R^5$ | représentent chacun, indépendamment les uns des autres, H ou un groupe alkyle en $C_{1-6}$, cycloalkyle en $C_{5-7}$, phényle, et $R^3$ et $R^4$ peuvent également former ensemble un pont alkylène en $C_{4-6}$, |
| m | est un nombre allant de 1 à 6, |
| p, q | représentent chacun, indépendamment l'un de l'autre, un nombre allant de 0 à 2, |

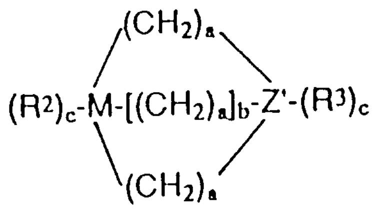

(II)

avec les significations précédentes pour M, $R^2$ et $R^3$ et dans laquelle

| | |
|---|---|
| Z' | représente N, P, |
| a | représente un nombre allant de 2 à 4, |
| b, c | représentent 0 ou 1, avec $b + c = 1$ , |

en tant que composants dans des systèmes catalytiques de coordination.

**2.** Utilisation selon la revendication 1, dans des systèmes catalytiques de coordination, pour la polymérisation par coordination et la métathèse d'alcènes et d'alcynes.

**3.** Systèmes catalytiques de coordination à base de composés contenant des métaux de transition appartenant aux groupes secondaires IV à VIII et de composés organométalliques du groupe principal III du tableau périodique des éléments, caractérisés en ce qu'ils contiennent au moins un composé de formule I ou II.

**4.** Procédé pour la préparation de polymères par polymérisation par coordination d'alcènes et/ou d'alcynes, caractérisé en ce que l'on utilise un système catalytique de coordination selon la revendication 3.

**5.** Procédé pour la préparation de composés hydrocarbonés insaturés par une réaction de métathèse catalysée d'alcènes ou d'alcynes, caractérisé en ce que l'on utilise, en tant que catalyseur de métathèse, un système catalytique de coordination selon la revendication 3.